# EUROPEAN PATENT SPECIFICATION

(11) **EP 3 275 312 B1**
(45) Date of publication and mention of the grant of the patent: **30.08.2023**
(21) Application number: 16768922.3
(22) Date of filing: 25.03.2016
(51) Int. Cl.: A01K 67/027, C12N 5/071, C12N 15/09

(54) **TRANSPLANT FISH**
TRANSPLANTATIONSFISCH
POISSON TRANSPLANTÉ

(30) Priority: 26.03.2015 JP 2015065152
(43) Date of publication of application: 31.01.2018
(73) Proprietor: National University Corporation Tokyo University of Marine Science and Technology, Tokyo 108-8477 (JP); Nippon Suisan Kaisha, Ltd., Minato-ku Tokyo 105-8676 (JP)
(72) Inventor: YOSHIZAKI, Goro, Tokyo 108-8477 (JP); TAKEUCHI, Yutaka, Tokyo 108-8477 (JP); YAZAWA, Ryosuke, Tokyo 108-8477 (JP); KAWAMURA, Wataru, Tokyo 108-8477 (JP)
(74) Representative: Winter, Brandl - Partnerschaft mbB
(86) International application number: PCT/JP2016/059551
(87) International publication number: WO 2016/153019

(56) References cited:
- WO-A1-2006/040926
- WO-A1-2009/118778
- WO-A1-2015/146184
- US-A1- 2012 304 322
- YAMAHA E. ET AL.: 'Developmental biotechnology for aquaculture, with special reference to surrogate production in teleost fishes' J. SEA RES. vol. 58, no. 1, 2007, pages 8 - 22, XP022138086
- HIROYUKI YOSHIKAWA ET AL.: 'Kaisangyo ni Okeru Dairi Shingyo Gijutsu no Kaihatsu: Nibeka Funensei Zasshu o Shukushu to shita Donor Yurai Jisedai no Seisan' THE JAPANESE SOCIETY OF FISHERIES SCIENCE SHUNKI TAIKAI KOEN YOSHISHU 27 March 2014, page 71, XP009506974
- YUTAKA TAKEUCHI: 'Ishukan Seigen Saibo Ishoku o Mochiita Ogata Shokuyo Kaisangyo Shubyo Seisan no Tei-energy-ka Gijutsu no Kaihatsu' SENTAN KENKYU JOSEI KIKIN JOSEIKIN (SAISENTAN.JISEDAI KENKYU KAIHATSU SHIEN PROGRAM) JISSHI JOKYO HOKOKUSHO (HEISEI 24 NENDO 16 January 2014, pages 1 - 4, XP009506979
- HIROYUKI YOSHIKAWA ET AL.: 'Jin'i Kozatsu ni yoru Nibeka Funensei Zasshu no Tansaku to Zasshu Shukushu no Dairi Shingyo Gijutsu eno Riyo' THE JAPANESE SOCIETY OF FISHERIES SCIENCE SHUNKI TAIKAI KOEN YOSHISHU 2013, page 150, XP009506973
- KAZUYA NASHIDA ET AL.: 'Masaba no yona Gomasaba' CHUO SUIKEN NEWS, [Online] 2005, pages 1 - 2, XP055494139 Retrieved from the Internet: <URL:HTTP://NRIFS.FRA.AFFRC.GO.JP/NEWS/NEWS 38/C/C.HTML> [retrieved on 2016-06-09]
- YUTAKA TAKEUCHI: 'Establishment of spermatogonial cell transplantation technique in marine teleost' BULLETIN OF THE JAPANESE SOCIETY OF SCIENTIFIC FISHERIES vol. 74, no. 4, 2012, pages 673 - 676, XP009506976
- YOSHIZAKI G ET AL: "Spermatogonial transplantation in fish: A novel method for the preservation of genetic resources", COMPARATIVE BIOCHEMISTRY AND PHYSIOLOGY. PART D: GENOMICS ANDPROTEOMICS, ELSEVIER, AMSTERDAM, NL, vol. 6, no. 1, 1 March 2011 (2011-03-01), pages 55-61, XP027589251, ISSN: 1744-117X [retrieved on 2010-05-15]
- XU KANG ET AL: "Development and application of biological technologies in fish genetic breeding", SCIENCE CHINA LIFE SCIENCES, ZHONGGUO KEXUE ZAZHISHE, CHINA, vol. 58, no. 2, 16 January 2015 (2015-01-16), pages 187-201, XP035442978, ISSN: 1674-7305, DOI: 10.1007/S11427-015-4798-3 [retrieved on 2015-01-16]

## Description

### TECHNICAL FIELD

The present invention relates to a transplant fish obtainable by a transplant fish production method.

### BACKGROUND ART

In recent years, the trend of declining fish hauls of natural fishing resources has continued, and accompanying this, the proportion of aquaculture production among total fish hauls has greatly increased. In aquaculture of marine fish, on the other hand, natural seed and fry are often developed to commercial product size. For this reason, the importance of resource management has increased and the introduction of complete aquaculture is more strongly demanded.

Complete aquaculture is an aquaculture style carried out without dependence on natural resources. Seed and fry are artificially produced from the fertilized eggs of a target fish species and the obtained artificial seed and fry are distributed as commercial products. Additionally, some individuals of the obtained artificial seed and fry are developed into parent fish, gametes are produced, and the obtained gametes are used in aquaculture. To achieve complete aquaculture, gametes with sufficient mass and weight to produce seed and fry must be obtained from the parent fish of the target fish species. Obtaining gametes sufficient for seed and fry production requires a breeding facility large enough to sustain parent fish as well as inducement of development and spawning using appropriate feed, and a great deal of space and labor are necessary. For this reason, it is said that there are few fish species established for use in seed and fry production to the extent that complete aquaculture can be easily introduced by many aquaculture enterprises.

Surrogate broodstock technology has gained attention as a technique that could solve this problem. This technology is a method whereby a target fish species for obtaining gametes is used as a donor, a fish species for producing gametes is used as a recipient, undifferentiated reproductive cells of the donor are transplanted into the recipient, and the recipient is used as surrogate broodstock. Examples of donor undifferentiated reproductive cells are primordial germ cells, spermatogonia, and oogonia. In other words, this is a technology for producing donor gametes by proliferating or differentiating donor reproductive cells in the gonads of a recipient and then producing next-generation individuals of the donor. In fish, male gametes have mobility and are called sperm, whereas female gametes lack mobility and are called eggs. In surrogate broodstock technology, to obtain sperm, the donor reproductive cells are transplanted into a male recipient, while, to obtain eggs, donor reproductive cells are transplanted into a female recipient.

As surrogate broodstock technology, JP-A-2003-235558 and Fisheries for Global Welfare and Environment, 5th World Fisheries Congress 2008 (2008) p. 209-219 describe a method for producing next-generation individuals of masu salmon (Oncorhynchus masou) using rainbow trout (Oncorhynchus mykiss) as surrogate broodstock. The method includes obtaining eggs and sperm of donor masu salmon by transplanting spermatogonia of masu salmon into recipient male and female rainbow trout, which is of the same family and genus.

Science Vol. 317 (2007) p. 1517 describes a method for producing next-generation individuals of masu salmon. The method includes producing triploid rainbow trout to obtain infertile surrogate broodstock that do not produce rainbow trout-derived sperm and eggs, and efficiently obtaining sperm and eggs of masu salmon by transplanting reproductive cells of masu salmon into the infertile surrogate broodstock. JP-A-2006-101845 also discloses a method for producing triploid masu salmon and transplanting rainbow trout-derived primordial germ cells into them. These publications state that, according to these methods, since there is substantially no formation of recipient-derived eggs or sperm, the complexity of selecting donor gametes from numerous recipient gametes is reduced and donor gametes can be obtained efficiently.

Fisheries Science Vol. 77 (2011) p. 69-77 describes that when yellowtail spermatogonia were transplanted into nibe croaker (Nibea mitsukurii), they were taken up into the gonads of the nibe croaker but did not attain gamete production. Biology of Reproduction Vol. 82 (2010) p. 896-904 describes that when spermatogonia of nibe croaker were transplanted into chub mackerel, it was ascertained that the nibe croaker reproductive cells were taken up into the chub mackerel gonads but did not attain gamete production.

Yoshizaki G. et al., Comparative Biochemistry and Physiology, Part D: Genomics and Proteomics, Elsevier, Amsterdam, NL, vol. 6, no. 1, 2011, p. 55-61 relates to "Spermatogonial transplantation in fish: a novel method for the preservation of genetic resources".

Xu Kang et al., Science China Life Sciences, Zhongguo Kexue Zazhishe, China, vol. 58, no. 2, 2015, p. 187-201 is directed to the "Development and application of biological technologies in fish genetic breeding".

### SUMMARY OF INVENTION

### Technical Problem

In surrogate broodstock technology developed up to now, fish species that can be used as surrogate broodstock are limited to those closely related to the donor, and the fish species to which surrogate broodstock technology can be applied are limited. No appropriate combinations of fish species for obtaining a sterile hybrid useful for surrogate broodstock technology are yet known.

The object of the present invention is to allow surrogate broodstock technology to be applied to donor fish species for which finding appropriate fish species to serve as surrogate broodstock was difficult in surrogate broodstock technology developed up to now, and to provide a hybrid fish species effective therefor.

### Solution to Problem

The present invention that achieves the above object is defined in the claims.

### EFFECTS OF INVENTION

The present invention allows surrogate broodstock technology to be applied to donor fish species of which finding appropriate fish species to serve as surrogate broodstock was difficult in surrogate broodstock technology developed up to now, and provides a hybrid fish species effective therefor.

### DESCRIPTION OF EMBODIMENTS

According to the present invention, a transplant fish is obtainable by a transplant fish production method comprising:
selecting a donor fish species;
selecting first and second fish species;
producing a sterile hybrid fish species of the selected first fish species and second fish species; and
transplanting reproductive cells of the donor fish species into the produced sterile hybrid fish species.

The hybrid fish species is usable in surrogate broodstock technology.

In the present invention, two different fish species, namely a first fish species and a second fish species which are a combination that could be sterile, are selected, and a sterile hybrid fish species is produced from these two specified species. Furthermore, in the present invention, a transplant fish is obtained by transplanting reproductive cells of a donor fish species into the produced sterile hybrid fish species, wherein the donor fish species is selected from the Scombridae family and wherein the first and second fish species are selected from species different from the donor fish species among species selected from the Sardini tribe, such as the Gymnosarda genus and Sarda genus; the Scomberomorini tribe, such as the Acanthocybium genus and Scomberomorus genus; the Scombrini tribe, such as the Scomber genus and Rastrelliger genus; and the Thunnini tribe, such as the Auxis genus, Euthynnus genus, Katsuwonus genus, and Thunnus genus. Since the hybrid fish species is sterile, gametes of the donor fish species can be efficiently produced in the transplant fish.

For example, since target gametes are not obtained in, for example, a hybrid fish species of yellowtail and nibe croaker, such a hybrid fish species cannot be used in surrogate broodstock technology. Artificial means such as sterilization treatment are required in order to obtain a sterile surrogate broodstock that can efficiently produce gametes of the donor fish species. Additionally, even for sterile surrogate broodstock obtained by sterilization treatment, efficiency in producing gametes of the donor fish species is not sufficient.

In contrast, in the present invention, it was found that there are combinations of fish species that can produce sterile hybrid fish species that can be used in surrogate broodstock technology. Thus, by selecting specified first fish species and second fish species for producing a hybrid fish species, a hybrid fish species that can be used in surrogate broodstock technology can be easily obtained. Furthermore, transplant fish and donor fish species gametes can be easily and efficiently obtained by using such a hybrid fish species. As a result, the range of surrogate broodstock that can be used in surrogate broodstock technology can be widened beyond the range previously imagined.

In the present specification, a donor fish species in surrogate broodstock technology is sometimes simply called a donor. In the present specification, a recipient fish species in surrogate broodstock technology is sometimes simply called a recipient.

In the present specification, a hybrid fish species is sometimes simply called a hybrid.

In the present specification, in addition to an independent step, the term "step" also refers to a step that achieves an intended object of the step even when the step cannot be clearly distinguished from other steps.

In the present specification, the numerical range indicated by "number to number" is taken to indicate a range including the respective numerical values before and after "to" as being the minimum and maximum values, respectively.

In a case in which the amount of a component type that may be included in the mixture is indicated herein, when there are plural substances corresponding to the component type in the mixture, the indicated amount means the total amount of the plural substances present in the mixture, unless specifically stated otherwise.

In the present invention, the transplantation step of transplanting reproductive cells of the donor fish species into the hybrid fish species and the steps subsequent to the transplantation step are performed according to surrogate broodstock technology.

Surrogate broodstock technology is a technique in which a target fish species of which gametes are to be obtained is used as a donor fish, and a fish species that is to produce gametes is used as a recipient fish; undifferentiated reproductive cells of the donor fish species are transplanted into the recipient fish species to obtain a transplant fish; by proliferating and differentiating the undifferentiated reproductive cells of the donor fish species in the gonads of the recipient fish species as the transplant fish, donor fish species-derived gametes are obtained in the body of the recipient fish species as the transplant fish, and seed and fry which are the offspring of the donor fish are obtained. In the present specification, "transplant fish" includes all targets that have received undifferentiated reproductive cells of the donor fish species. These targets may be at any stage of development and growth, including embryos, larvae, young fry, adult fish, and the like. The transplant fish may be at any stage after transplant, provided that they possess received undifferentiated reproductive cells or matured reproductive cells or gametes thereof. The term "seed and fry" refers to the larvae and young fry of the targeted fish.

Examples of undifferentiated reproductive cells used in surrogate broodstock technology are primordial germ cells, spermatogonia, and oogonia. To obtain sperm, donor reproductive cells are transplanted into a male recipient, while, to obtain eggs, donor reproductive cells are transplanted into a female recipient. As gonads, testicles may be used when sperm are to be obtained, and ovaries may be used when eggs are to be obtained. Donor reproductive cells transplanted into a male recipient may give rise to donor sperm by proliferation and differentiation in the testicles of the recipient. Donor reproductive cells transplanted into a female recipient are expected to give rise to donor eggs by proliferation and differentiation in the ovaries of the recipient. Fertilized eggs may be obtained by cross-fertilizing sperm or eggs obtained in this manner, or by cross-fertilizing separately obtained eggs or sperm. By developing the obtained fertilized eggs, seed and fry of the target fish species may be obtained.

The donor is, but not limited to, from the perspective of economic utilization value, farmed fish, marine fish, or farmed and marine fish are preferably used as the donor fish species. Donors include edible fish species, fish species for materials harvesting, and prized fish species. According to the present invention the donor fish species is selected from the Scombridae family.

The donor undifferentiated reproductive cells used in surrogate broodstock technology include primordial germ cells obtained from gonads prior to sex differentiation, spermatogonia obtained from testicles, oogonia obtained from ovaries, and the like. These undifferentiated reproductive cells may be used as a mixture of cells at different stages of differentiation or as cells of a specified stage of differentiation selected from the perspective of ease of uptake into the gonads.

Undifferentiated reproductive cells may be obtained from a donor by harvesting using a customary method from donor tissue in accordance with the stage of differentiation of the target undifferentiated reproductive cells. For example, undifferentiated reproductive cells may be obtained by resecting gonads prior to sex differentiation or tissue after sex differentiation, for example, testicles or ovaries, from the donor, and then physically separating them or treating them with a protease or the like to disperse them into individual cells from the tissue. Individual dispersed cells may be isolated using, for example, a marker antibody or a cell sorter.

Undifferentiated reproductive cells may be obtained from cryopreserved matter or live individuals. To increase the success rate of surrogate broodstock technology, it is preferred that the undifferentiated reproductive cells be obtained from live fish. As cryopreserved matter, that obtained by freezing in units of individuals, organs, and cells may be used.

Freezing of cells is preferably performed using a suitable cryopreservative. Examples of suitable cryopreservatives include polyethylene glycol, ethylene glycol, carboxymethylcellulose, dimethylsulfoxide (DMSO), glycerol, glucose, trehalose, lactose, mannitol, dextran, dextran sulfate, pullulan, fetal bovine serum (FBS), bovine serum albumin (BSA), fish egg yolk, skim milk, and the like. The concentration (content) of cryopreservative may be selected as appropriate depending on the type of cryopreservative used. For example, a concentration of from 10 mM to 500 mM may be used for sugars such as glucose, trehalose, dextran, and pullulan, from 0.1 M (mol/L) to 10 M may be used for DMSO and the like, and from 0.1 M to 10 M may be used for ethylene glycol, propylene glycol, and the like. These cryopreservatives may be used individually or in combinations of two or more types.

The step of harvesting primordial germ cells preferably includes ascertaining that the obtained cells are primordial germ cells using a primordial germ cell marker. Markers that can be used to ascertain that they are primordial germ cells include the vasa gene, the CD205 gene, and the like. It can be ascertained by checking expression, modification, or localization of gene or gene product, or a combination of two or more of these.

To ascertain gene expression, it is easy to check mRNA transcribed from the specified gene by polymerase chain reaction (PCR). To ascertain gene expression and localization, it is easy to check RNA transcribed from the specified gene by in situ hybridization. To ascertain gene product expression, modification, and localization, it is easy to check a gene product obtained by expressing and translating from the specified gene using an antibody specific to that gene product.

When introducing donor cells into a recipient, it is preferable to introduce them into an embryo or an individual in the larval stage before the immune system of the recipient is fully functioning. Introduction may be performed using a manipulator such as a micromanipulator, electric scalpel, laser scalpel, or the like. Donor cells may be introduced into any tissue or site of the recipient. Examples of the tissue or site as the target of introduction include the epidermis and the peritoneum.

The number of cells introduced into an embryo or an individual in the larval stage is, but not limited to, and may be, for example, from 1 to 200000 cells. The number of cells introduced into an embryo or an individual in the larval stage may be selected as appropriate based on the size of the embryo or individual.

Engraftment of the donor reproductive cells in the recipient gonads may be ascertained using a marker that can differentiate between donor cells and recipient cells. Markers that can be used include genes, antibodies, fluorescent dyes, and the like, and these markers may be used individually or in combinations of two or more types. Genes that may serve as markers are genes expressed in the undifferentiated reproductive cells, examples of which include the vasa gene, dead-end gene, and the like. Fluorescent dyes that may serve as markers include PKH26, CFSE, and the like.

When a gene is used, PCR is performed with a primer designed using a site of the marker gene where the sequence differs between the donor and the recipient. Alternatively, checking is easy by in situ hybridization using a site where the sequence differs between the donor and the recipient. Engraftment may be ascertained under a fluorescence microscope by labeling the donor reproductive cells with a fluorescent dye prior to introduction. Engraftment may be ascertained immediately after introduction. Engraftment is preferably ascertained not less than 7 days after introduction in order to ascertain whether or not they were engrafted in the recipient and are functioning as cells. Donor sperm or eggs may be obtained from individuals in which engraftment in the recipient gonads was observed 7 days after introduction.

A hybrid means an individual also having a genome derived from a different strain, lineage, or biological species in its cells. It includes individuals produced by crossbreeding, cell fusion, or gene transfer, or a combination of two or more of these. In fish, different breeding populations biologically separated by habitat or the like are generally recognized as different strains, lineages, or biological species, but there are cases in which crossbreeding between breeding populations occurs in adjacent regions where different breeding populations are distributed, and so-called hybrids occur. However, in the natural environment, it is not well understood under which circumstances crossbreeding occurs and hybrids are obtained. Hybrids also include individuals produced artificially. Hybrids may also be similarly artificially produced in cases where a strain or lineage has been produced and sustained.

When an individual as a hybrid is produced by crossbreeding, a crossbreed may be obtained by cross-fertilizing individuals of different strains, lineages, or biological species.

When an individual as a hybrid is produced by cell fusion, a method of fusing eggs or separated germ cells of different species may be used. In cell fusion, cells may be directly fused to each other using a cellular membrane fusion agent, electrical stimulation, photic stimulation, or thermal stimulation, or a combination of these. An example of a cellular membrane fusion agent that may be used is polyethylene glycol. The hybrid cells obtained by cell fusion sometimes retain all chromosomes and sometimes retain only a portion of chromosomes. In the present invention, a genome required for the transplanted reproductive cells to function as egg or sperm when donor undifferentiated reproductive cells have been transplanted may be assembled.

When an individual as a hybrid is produced by gene transfer, it can be produced by transferring chromosomes or genes of one species into an egg of another species. The transferred chromosomes may be introduced in any amount provided that it is an amount required for the transplanted reproductive cells to function as egg or sperm when donor undifferentiated reproductive cells have been transplanted into the hybrid serving as the recipient. For example, one or a plurality of chromosomes or a partial fragment thereof may be transferred. An appropriate chromosome that functions after transplantation may be found by introducing a combination of different chromosomes.

As for transferred genes, one or two or more genes may be introduced regardless of which genes they are, provided that they are genes required for the transplanted reproductive cells to function as egg or sperm when donor undifferentiated reproductive cells have been transplanted into the hybrid serving as the recipient. An example of such a gene is the gsdf gene.

As the hybrid used as the recipient, a strain, lineage, or biological species having properties suitable for a recipient is preferably produced using the first fish species and the second fish species. Examples of properties suitable for a recipient include high survival rate in an artificial breeding environment, small size of required breeding facility, low equipment cost for required temperature regulation, low cost of food, ease of harvesting eggs or sperm, and the like. Hybrids produced based on this method preferably inherit properties suitable for a recipient. To ensure that the produced hybrid will inherit properties suitable for a recipient, it is preferred that the hybrid genome contain a greater amount of the genome of the strain, lineage, or biological species having properties suitable for a recipient. Having a greater amount of the genome of the strain, lineage, or biological species having properties suitable for a recipient can be ensured by artificially ploidizing eggs obtained from the strain, lineage, or biological species having properties suitable for a recipient.

In the hybrid, the proportion of the genome derived from the donor or a species closely related to the donor relative to the total genome (referred to as "donor or the like" hereafter in this paragraph) may be reduced in order to further increase the properties advantageous for surrogate broodstock. For example, the hybrid may have not greater than one-half of the genome of the donor or the like per cell, or may have not greater than one-third of the genome of the donor or the like per cell. The amount of genome of the donor or the like in the hybrid may be set to an amount calculated by DNA flow cytometry by selecting somatic cells or blood cells as the cell of interest, and using cells harvested by a conventional method. For example, in the case of a hybrid cell having one set each of the genome of the donor or the like and the genome of a fish species other than the donor or the like, the amount of genome of the donor or the like in the hybrid cell is one-half. When a chromosome derived from the donor or the like is missing or DNA derived from the donor or the like is defective, or when it is a hybrid cell produced by transferring a portion of the chromosomes or DNA of the genome derived from the donor or the like into the genome of a fish species other than the donor or the like, the amount of genome of the donor or the like is not greater than one-half. In the case of a hybrid cell having one set each of the genome of the donor or the like and the ploidized genome of a fish species other than the donor or the like, the amount of genome of the donor or the like in the hybrid cell is one-third. When a chromosome derived from the donor or the like is missing or DNA derived from the donor is defective, or when it is a hybrid cell produced by transferring a portion of the chromosomes or DNA of the genome derived from the donor into the genome of a fish species other than the donor or the like, the amount of genome of the donor or the like can be ascertained to be not greater than one-third.

The first and second fish species selected for producing the hybrid fish species that can be used as the surrogate broodstock are fish species which are a combination that could be sterile. Such a combination of first and second fish species is a combination that produce a sterile fish species that can be used as surrogate broodstock. In other words, the obtained hybrid fish species has the capability of having reproductive cells of the donor fish species engrafted in the gonads thereof and the capability of producing gametes and be sterile.

An example of the combination that the obtained hybrid fish species has the capability of having reproductive cells of the donor fish species engrafted in its gonads and the capability of producing gametes and is sterile includes a combination of species capable of forming an individual that is capable of forming organs and tissues sufficiently to differentiate and grow primordial germ cells into gametes, and, however, lacks sufficient capability to produce primordial germ cells.

It is surmised that when a hybrid is produced from a first fish species and a second fish species having the above relationship, differentiation and proliferation of the reproductive cells of the hybrid itself do not function sufficiently, and as a result, its own reproductive cells are not produced in the gonads; that is, potential reproductive cells are destroyed or lost. On the other hand, it is surmised that in a hybrid produced from a first and second fish species having the above relationship, the functions of organs such as the gonads are not lost, and as a result, when reproductive cells of the donor fish species are transplanted from externally, production of gametes of the donor fish species in the gonads is possible without harming differentiation and proliferation of the transplanted reproductive cells of the donor fish species.

Examples of first and second fish species that can achieve such a combination include combinations of species whose habitats overlap and crossbreeding is observed but are considered different species taxonomically, and combinations resulting in malfunction on a cellular biological level when their reproductive cells develop. The relationship which is mentioned below between the first and second fish species may also be satisfied by overlapping two or more different species.

When the first and second fish species are a combination of species whose habitats overlap and crossbreeding is observed but are considered different species taxonomically, it is thought that some sort of reproductive separation mechanism exists and the combination is unlikely to result in gametes that can be cross-fertilized, in order to preserve them as different species despite the fact that their habitats overlap and crossbreeding has been confirmed.

Examples of combinations having a relationship based on habitat and taxonomic ranking include combinations of species that are both of different species of the same family as the donor fish. Examples include a first fish species that is a different species of the same family as the donor fish and has an overlapping habitat, and a second fish species that is a different species of the same family as the donor fish and has an overlapping habitat, and that is a different species of the same family and genus as the first fish species. With this combination, since the first and second fish species are both of the same family as the donor, it can be expected that the transplanted reproductive cells of the donor will function well in the produced hybrid. Furthermore, when donor reproductive cells are transplanted into the produced hybrid, the donor reproductive cells are readily engrafted and donor gametes are readily obtained. In the case of a fish species which is a different species of the same family as the donor fish and also has an overlapping habitat, it is surmised that sterility tends to occur because they are taxonomically separate to a suitable degree due to the fact that they are fish species that continue to exist as different species despite having a common dwelling environment, namely having an overlapping habitat with the donor.

The first fish species and the second fish species in this combination may be selected from any species different from that of the donor but in the same family. Specifically, the first fish species and second fish species may be selected from a different species in the same family, tribe, and genus, a different species in a different genus of the same family and tribe, and a different species of a different tribe and genus of the same family as the donor.

According to the present invention, the donor is a fish species of the Scombridae family, the first fish and the second fish are selected from species different from the donor among species belonging to various tribes such as the Sardini tribe, such as the Gymnosarda genus and Sarda genus; the Scomberomorini tribe, such as the Acanthocybium genus and Scomberomorus genus; the Scombrini tribe, such as the Scomber genus and Rastrelliger genus; and the Thunnini tribe, such as the Auxis genus, Euthynnus genus, Katsuwonus genus, and Thunnus genus.

When the donor is, for example, Pacific bluefin tuna of the Thunnus genus of the Thunnini tribe of the Scombridae family, at least one of the first fish species and the second fish species may be a species of the same family, tribe, and genus as the donor, namely, a species other than Pacific bluefin tuna that is a member of the Thunnus genus of the Thunnini tribe of the Scombridae family, or a species of a different genus of the same family and tribe, namely, a species of a genus other than Thunnus of the same family and tribe. Examples of fish species in the Thunnus genus of the Thunnini tribe of the Scombridae family other than Pacific bluefin tuna include albacore tuna, yellowfin tuna, bigeye tuna, and the like. Examples of fish species of the Thunnini tribe of the Scombridae family other than of the Thunnus genus include Katsuwonus pelamis, Euthynnus affinis, Euthynnus alletteratus, Auxis thazard, and Auxis rochei.

A first fish species and second fish species of mutually the same family and genus may be selected from fish species of mutually different species of the same genus. For example, when the donor is the Pacific bluefin tuna of the Thunnus genus of the Thunnini tribe of the Scombridae family, examples of the combination of first and second fish species include combinations of different species of the Thunnus genus of the Thunnini tribe of the Scombridae family, such as albacore tuna and bigeye tuna; combinations of different species of the Euthynnus genus of the Thunnini tribe of the Scombridae family, such as Euthynnus affinis and Euthynnus alletteratus, Euthynnus affinis and Euthynnus lineatus, and Euthynnus alletteratus and Euthynnus lineatus; combinations of different species of the Auxis genus of the Scombridae family, such as Auxis thazard and Auxis rochei; and combinations of different species of the Scomber genus of the Scombridae family, such as chub mackerel and blue mackerel; and the like.

A first fish species and second fish species of mutually the same family and tribe may be selected from fish species of a mutually different genus of the same tribe. For example, when the donor is the Pacific bluefin tuna of the Thunnini tribe of the Scombridae family, examples of the combination of first and second fish species include combinations of the Euthynnus genus and Thunnus genus, the Euthynnus genus and the Auxis genus, and the Euthynnus genus and the Katsuwonus genus and the like, which are in the Thunnini tribe of the Scombridae family.

Examples of combinations of first and second fish species resulting in deficient function on a cellular biological level when their reproductive cells develop include combinations of species in which the centromere positions on corresponding chromosomes differ. For example, when a metacentric or submetacentric chromosome in one species corresponds to an allocentric chromosome in the other species, they cannot be correctly paired in the centromere during fission, resulting in chromosome non-separation or a partial deletion, overlap, reversal, or translocation of chromosomes, such that a portion of genetic information does not function during development of the crossbreed, resulting in malformation of reproductive cells. Incorrect pairing of centromeres tends to occur during meiosis in particular. When the number of chromosomes decreases in meiosis, the centromere sites become smaller, which tends to cause chromosome non-separation or a partial deletion, overlap, reversal, or translocation of chromosomes. For this reason, reproductive cell defects tend to occur in a crossbreed that is sterile due to chromosome non-separation. Thus, for example, in the Scombridae family, when species having different centromere positions are combined, a hybrid with malformed reproductive cells could be produced.

Examples of combinations of first and second fish species resulting in malfunction on a cellular biological level when their reproductive cells develop include combinations in which correct conservation or function expression of mitochondria is difficult. Mitochondria perform maternal inheritance and are passed down only from the mother. Fish having the property of migrating the ocean needs to move for a long time because their swimming distance is long. They have red muscle flesh possessing a large number of mitochondria. On the other hand, fish that inhabit coastal areas have a short swimming distance but require instantaneous force since they often encounter enemies. They have white muscle flesh possessing few mitochondria. When such fish species are combined, it tends to result in an individual lacking proper control of the number, fission, function conservation, or distribution of mitochondria in the cells, and some cells do not function during crossbreed development, resulting in malformation of reproductive cells. When mitochondrial control deficiency is significant, it hinders development of the individual itself, but when mild, it results in a phenotype of reproductive cell deficiency. In spermatogenesis, in particular, mitochondria may induce tail elongation of sperm, and a mitochondrial control abnormality will result in spermatogenesis failure. Thus, for example, in the Scombridae family, when species having different flesh colors are combined, a hybrid with malformed reproductive cells could be produced.

Examples of other combinations of first and second fish species resulting in malfunction on a cellular biological level when their reproductive cells develop include combinations in which resetting of DNA methylation, histone methylation and acetylation, and the like is difficult. DNA methylation and histone methylation and acetylation influence the expression of a variety of genes. Furthermore, it is known that cross-fertilization between different species induces large-scale changes in DNA methylation and histone methylation and acetylation patterns, and therefore influences the expression or function of various genes and could result in malformation of reproductive cells.

Examples of other combinations of first and second fish species resulting in malfunction on a cellular biological level when their reproductive cells develop include combinations in which transcription factor DNA binding patterns differ and correct gene expression is difficult. It is known that the mRNA transcription pattern changes in crossbreed formation, and this influences the expression of a variety of genes. As a result, it influences the function of a variety of genes and can result in malformation of reproductive cells.

Examples of other combinations of first and second fish species resulting in malfunction on a cellular biological level when their reproductive cells develop include combinations in which meiosis is difficult and ploidizing can occur, and combinations in which function expression of factors that suppress transposons or viruses is incomplete. There are known examples assumed to be caused by transposons, virus-like factors, or viruses having been inactivated in species-specific repetitive sequences seen on chromosomes, in which activation occurs by cross-fertilization of different species. This activation of transposons, virus-like factors, or viruses influences the expression or function of various genes, resulting in malformation of reproductive cells. For example, it is known that sterility results from an abnormality in piRNA in zebra fish and carp.

When the donor is the Pacific bluefin tuna, specific combinations of first and second fish species resulting in malfunction on a cellular biological level when their reproductive cells develop include a combination of albacore tuna and bigeye tuna, a combination of Euthynnus affinis and Euthynnus alletteratus, a combination of Euthynnus affinis and Euthynnus lineatus, a combination of Euthynnus alletteratus and Euthynnus lineatus, a combination of Auxis thazard and Auxis rochei, and a combination of chub mackerel and blue mackerel.

The hybrid production method and the transplant fish production method are particularly effective for enabling artificial production of hybrids in marine fish in which formation of hybrids has not often been seen in nature.

For example, in the case of char of the Salvelinus pluvius of the Salmonidae family, since food ingestion is poor in artificial breeding, the survival rate is low, and methods for harvesting eggs or sperm have not been sufficiently established. Breeding methods for Salvelinus malma in the same genus as char have not been established, either. In the case of Seriola quinqueradiata of the Carangidae family, large equipment is required for breeding, and temperature regulation for harvesting eggs or sperm is costly. Seriola dumerili and Seriola lalandi in the same genus as Seriola quinqueradiata are not considered suitable as surrogate broodstock for the same reason. Pacific bluefin tuna of the Thunnus genus of the Scombridae family is not considered suitable as surrogate broodstock from the perspectives that large equipment is required for breeding and that breeding for three or more years is required to harvest eggs or sperm, Southern bluefin tuna, bigeye tuna, albacore tuna, longtail tuna, and yellowfin tuna, which are fish species in the Thunnus genus closely related to Pacific bluefin tuna, also require large equipment for breeding, and methods for harvesting eggs or sperm have not been established.

In such species, it is preferred that a hybrid produced from the first and second fish species specified in this method be used as a recipient.

The produced hybrid is an individual having the property that gametes of the recipient hybrid itself are difficult to produce or having the property of so-called sterility, in which reproductive cell function is insufficient but gonad function is sufficient.

Such individuals may be for example, specified as individuals that exhibit a phenotype of reproductive cell deficiency. Examples of phenotypes of reproductive cell deficiency include phenotypes in which a reproductive cell marker is negative but a gonad cell marker is positive. Examples of reproductive cell markers include the vasa gene, dead-end gene, and the like. Examples of gonad markers include the gsdf gene and the like. To ascertain gene expression, it is easy to check mRNA transcribed from the specified gene by PCR. To ascertain gene expression and localization, it is easy to check RNA transcribed from the specified gene by in situ hybridization. To ascertain gene product expression, modification, and expression, it is easy to check a gene product obtained by expressing and translating from the specified gene using an antibody specific to that gene product. Since such a hybrid has functional gonads and produces few or no hybrid-derived gametes, donorderived gametes can be efficiently obtained. Furthermore, such a hybrid is very useful as a hybrid for surrogate broodstock technology.

The produced hybrid is used as a recipient of donor reproductive cells. Specifically, after the produced hybrid is reared over the required period depending on the case, donor reproductive cells are transplanted into this hybrid as a recipient. The method for transplanting the donor reproductive cells into the hybrid is as described above. By transplanting the donor reproductive cells into the hybrid, a transplant fish is produced. This transplant fish is useful in that gametes can be obtained from the donor reproductive cells with good productivity.

The fish gamete production method includes obtaining a transplant fish by the above transplant fish production method, and obtaining donor fish-derived gametes from the obtained transplant fish.

Donor fish-derived gametes, namely eggs or sperm, can be obtained from the transplant fish by inducing maturing gametes and harvesting them by conventional methods according to the fish species. In particular, since the hybrid is sterile, recipient-derived eggs and sperm are not produced, donorderived eggs and sperm in the transplant fish can be easily obtained.

Eggs and sperm produced in the hybrid can be obtained by rearing the produced hybrid, transplanting donor primordial germ cells using this hybrid as a recipient, and producing a transplant fish. Primordial germ cells from the transplanted donor enter the gonads of the recipient and give rise to donorderived eggs or sperm. The eggs or sperm in the transplant fish can be isolated or harvested by known methods such as abdominal squeezing.

The transplant fish according to another embodiment of the present invention is one that can be produced using the transplant fish production method disclosed above.

Since the reproductive cells of a donor fish species for which finding appropriate fish species to serve as surrogate broodstock was difficult up to now have been transplanted into this transplant fish, the reproductive cells of the donor fish species can be grown into gametes in the transplant fish. As a result, gametes of donor fish of which seed and fry production was particularly difficult up to now can be obtained with good productivity.

The seed and fry production method disclosed herein includes obtaining a transplant fish by the above transplant fish production method, and obtaining seed and fry from the obtained transplant fish.

In the step of obtaining seed and fry from the transplant fish, the type of other gametes used to cross-fertilize with the gametes produced in the transplant fish are not particularly limited.

For example, seed and fry in the present invention can be obtained by rearing the produced hybrid, and cross-fertilizing eggs or sperm from a transplant fish obtained by transplanting donor primordial germ cells into the hybrid as a recipient with sperm or eggs obtained from another individual to obtain fertilized eggs, and rearing the fertilized eggs. The seed and fry in the present invention can also be obtained by rearing the produced hybrid, and cross-fertilizing eggs or sperm obtained by transplanting donor primordial germ cells into the hybrid as a recipient with sperm or eggs obtained from the donor. They can also be obtained by rearing the produced hybrid, and cross-fertilizing eggs or sperm obtained by transplanting donor primordial germ cells into the hybrid as a recipient with themselves. By so doing, seed and fry of a donor fish species of which seed and fry production was difficult up to now as well as seed and fry possessing the genes of the donor fish species can be provided.

The adult fish production method in the present invention includes obtaining a transplant fish by the above transplant fish production method, obtaining seed and fry from the obtained transplant fish, and rearing the obtained seed and fry to adulthood. The present invention encompasses adult fish obtained by this method. As a result, it is easier than in the past to provide adult fish of a donor fish species of which seed and fry production was difficult up to now. The obtained adult fish can be widely used as farmed fish.

The transplant fish and the hybrid for surrogate broodstock technology, the sperm or eggs obtained from the transplant fish, the seed and fry obtained from the transplant fish, and the adult fish obtained by growing the seed and fry obtained from the transplant fish are useful in, for example, breeding of farmed fish.

### EXAMPLES

The present invention is described below in detail using examples. However, the present invention is not limited in any manner by these examples. Furthermore, unless indicated otherwise, "%" in the below described examples is taken to mean "wt.%".

### Example 1

### (1) Production of crossbred mackerel

As a hybrid, a crossbred mackerel of blue mackerel and chub mackerel, that is, a blue mackerel-chub mackerel hybrid, was produced by artificial fertilization using unfertilized eggs obtained by squeezing mature female blue mackerel and sperm harvested from mature male chub mackerel. The fertilized eggs of the obtained blue mackerel-chub mackerel hybrid were reared in 17°C to 18°C seawater. The fertilization rate was measured 1 to 2 hours after fertilization, the embryo formation rate was measured 15 hours after fertilization, and the hatching rate was measured 2 days after fertilization.

Furthermore, the fertilization rate, embryo formation rate, and hatching rate of blue mackerel fertilized eggs obtained by artificial fertilization of unfertilized eggs of wild-type blue mackerel and sperm of wild-type blue mackerel were measured, and the fertilization rate, embryo formation rate, and hatching rate of low-temperature-treated blue mackerel fertilized eggs were also measured. These were compared with those of the blue mackerel-chub mackerel hybrid. The results are shown in Table 1. The blue mackerel low-temperature treatment was triploid treatment, and was performed for 10 minutes at 3°C at 5 minutes after fertilization. The triploid rate was 100%.

**[Table 1]**

| | Fertilization rate (%) | Embryo formation rate (%) | Hatching rate (%) |
|---|---|---|---|
| Untreated section (blue mackerel diploid) | 45.0±2.7 | 24.2±2.7 | 17.5±2.5 |
| Low-temperature treated section (blue mackerel triploid) | 38.3±6.1 | 5.8±2.0 | 4.2±2.0 |
| Blue mackerel-chub mackerel hybrid | 27.5±5.5 | 15.0±3.4 | 12.5±1.7 |

As shown in Table 1, the embryo formation rate and hatching rate of the blue mackerel-chub mackerel hybrid were slightly lower than those of the wild-type but were higher than those of the low-temperature treatment group. Thus, it was understood that blue mackerel-chub mackerel hybrid fish can be produced stably and in large quantity compared to infertile fish triploidized by low-temperature treatment.

The hatched larvae of the obtained blue mackerel-chub mackerel hybrid were subjected to species identification using "Mackerel Checker-I kit" available from SCOTS (Science Center of Tensei-Suisan, Tensei Suisan Co., Ltd) and to PCR-RFLP using the mitochondrial cytochrome b gene region (cytB region) as a target. PCR-RFLP was performed according to Fish Identification Manual for the Mackerel Genus (Food and Agricultural Material Inspection Center, Independent Administrative Agency), Fisheries Research Agency, Independent Administrative Agency)). As a result, a crossbreed obtained as a result of fertilization of a blue mackerel egg and a chub mackerel sperm, that is, a blue mackerel-chub mackerel hybrid was ascertained. This blue mackerel-chub mackerel hybrid grew to a body length of 32 cm and a body weight of 460 g in approximately 2 years. Table 2 shows the body measurement data of the blue mackerel-chub mackerel hybrid and chub mackerel. In Table 2, "GSI" is the gonadosomatic index, represented by the equation [gonad weight (mass) × 100]/body weight.

**[Table 2]**

| | Body length (cm) | Body weight (g) | Gonad mass (g) | GSI (%) |
|---|---|---|---|---|
| Blue mackerel-chub mackerel hybrid | 31.8 | 461.2 | 0.232 | 0.05 |
| Chub mackerel | 29.5 | 415 | 45 | 10.84 |

### (2) Characteristics of blue mackerel-chub mackerel hybrid

Reproductive capability could not be ascertained in any of the above obtained blue mackerel-chub mackerel hybrids at 30 days old, 60 days old, or 120 days old. The reasons for this were examined as described below based on tissue staining and gene expression.

Tissue sections of the gonads of the above obtained blue mackerel-chub mackerel hybrids at 30 days old, 60 days old, or 120 days old were produced, and the gonads were observed by tissue staining with hematoxylin and eosin (HE) stain. As a result, undifferentiated gonads were present in the peritoneum of the young fry, and the presence of reproductive cells was also ascertained. The shape of the undifferentiated gonads was similar to that of a normal chub mackerel.

It is known that at 60 days of age, 50% each of males and females are generally observed in chub mackerel, and ovaries and testicles can be identified. It is also known that at 120 days of age, the presence of oocytes can be ascertained in the ovaries.

On the other hand, in the blue mackerel-chub mackerel hybrid, the presence of reproductive cells was no longer ascertained at 60 days of age. At 120 days of age, although the gonads exhibited a testicle-like shape in 100% of individuals, the presence of reproductive cells was not ascertained. Thus, in the blue mackerel-chub mackerel hybrid, all individuals had male-type gonads and exhibited a strong trend toward becoming male.

Furthermore, in the blue mackerel-chub mackerel hybrid, expression of the vasa gene, which is a molecular marker of reproductive cells, was examined by PCR.

First, total RNA was extracted from the gonads of the blue mackerel-chub mackerel hybrid using ISOGEN (Nippon Gene Co., Ltd.). cDNA was synthesized using SuperScript III First-Strand Synthesis System (Life Technologies Corp.) with 5 µg of the total RNA as a template. The obtained cDNA was diluted 25-fold and used as a template, and a total of 10 µL of reaction solution was prepared, containing the above diluted cDNA as well as 2.5 units of TaKaRa Ex Taq (Takara Bio Inc.), 1 µL of 10X Ex Taq Buffer, 0.2 mM of dNTP Mixture, and 1 µM each of a forward primer (CTATTTGTTCCTGGCTGTGG: SEQ ID NO: 1) and a reverse primer (GCAGACTCTTCTAACCATGAAGG: SEQ ID NO: 2). After this reaction solution was incubated for 3 minutes at 94°C using a thermal cycler, 30 cycles consisting of 30 seconds at 94°C, 30 seconds at 58°C, and 1 minute at 72°C were repeated, and finally, it was incubated for 3 minutes at 72°C.

As a result, the expected amplification of the 528 bp chub mackerel vasa gene sequence (accession no. GQ404693, 1332-1859) and the 528 bp blue mackerel vasa gene sequence (accession no. GU581279, 1371-1898) were not observed, and no expression of either the chub mackerel vasa gene or the blue mackerel vasa gene was detected in the gonads of the blue mackerel-chub mackerel hybrid.

Additionally, to ascertain the expression distribution of various markers in the gonads of the blue mackerel-chub mackerel hybrid, in situ hybridization was performed using the vasa gene and the gsdf gene, which is a gonad cell marker.

In situ hybridization was performed using a 528 bp blue mackerel vasa gene probe (accession no. GU581279, 1371-1898) or a 488 bp chub mackerel gsdf gene probe (accession no. GQ404694, 1-488). The series of experimental operations in the in situ hybridization were performed according to Developmental Biology vol. 301 (2007) pp. 266-275.

As a result, vasa expression cells were not observed in the gonads of the blue mackerel-chub mackerel hybrid but it was ascertained that gsdf expression cells were distributed in the same manner as in a normal blue mackerel. In wild-type chub mackerel and blue mackerel, substantially all individuals generally reach maturity at 2 years of age, but in the blue mackerel-chub mackerel hybrid, immature testicle-like gonads without reproductive cells remained unchanged.

Thus, these blue mackerel-chub mackerel hybrids were sterile fish lacking reproductive cells.

Furthermore, blue mackerel-chub mackerel hybrids obtained using chub mackerel eggs and blue mackerel sperm were similarly ascertained to be sterile fish lacking reproductive cells. At 2 years of age, when normal chub mackerel mature and spermiate, it was ascertained by tissue observation by HE staining of the gonads that no reproductive cells were present in the blue mackerel-chub mackerel hybrid.

### Example 2

### (1) Transplantation of spermatogonia of Pacific bluefin tuna into blue mackerel-chub mackerel hybrid

As donor cells, dispersed cells harvested from testicles of a 30 kg immature male Pacific bluefin tuna were used. Pacific bluefin tuna testicles harvested from Pacific bluefin tuna and cooled with ice overnight were cut into 1 mm squares, and reacted in L-15 medium "Leibovitz's L-15" (Life Technologies Corp.) containing collagenase (2 mg/mL), Dispase II (500 U/mL), fetal bovine serum (5 v/v%), and DNase (100 U/mL), and a cell suspension was obtained. The cell membranes of cells contained in this suspension were stained with red fluorescent dye PKH26 and then transplanted into the peritoneum of hosts using an injector in a quantity of 10000 to 100000 cells per host.

Blue mackerel-chub mackerel hybrid larvae produced in Example 1 at 10 days after hatching were used as hosts. Furthermore, normal chub mackerel diploids and chub mackerel triploids were produced and used as hosts. The chub mackerel triploids were obtained by incubating chub mackerel fertilized eggs for 20 minutes in 3°C seawater 7.5 minutes after fertilization.

Transplantation was carried out in 350 blue mackerel-chub mackerel hybrid larvae, 50 chub mackerel diploids, and 200 chub mackerel triploids. The host fish after transplantation were housed in a 100 L Sunlight water tank (Tanaka Sanjiro Co., Ltd.) placed in a water bath set to 18°C, and were bred for approximately 1 month. After that, they were transferred to a 5000 L FRP water tank (long round type, DF-5100S, earth) and further bred in 20°C seawater. Five months after transplantation, 67 (19.1%) of the blue mackerel-chub mackerel hybrid hosts survived, and the surviving individuals reached a full length of 18 cm (body weight 50 g). Furthermore, 19 (38.0%) of the chub mackerel diploids survived, and the surviving individuals reached a full length of 19 cm (body weight 53 g). Twenty-two (11.0%) of the chub mackerel triploid hosts survived, and the surviving individuals reached a full length of 19 cm (body weight 61 g).

### (2) Characteristics of transplant fish

Among the obtained individuals, gonads were harvested from 40 chub mackerel-blue mackerel hybrids, 19 chub mackerel diploid hosts, and 13 chub mackerel triploid hosts. Total RNA was extracted from the gonads and subjected to RT-PCR using a primer (manufactured by Operon Corp.) specific to the Pacific bluefin tuna vasa gene.

First, similar to Example 1, total RNA was extracted from the gonads of blue mackerel-chub mackerel hybrid hosts, chub mackerel diploid hosts, and chub mackerel triploid hosts using ISOGEN (Nippon Gene Co., Ltd.), and cDNA was synthesized with this as a template. Each of the obtained cDNA were used as templates, and a total of 10 µL of reaction solution was prepared, containing each template as well as 2.5 units of TaKaRa Ex Taq (Takara Bio Inc.), 1 µL of 10X PCR Buffer, 0.2 mM of dNTP Mixture, and 1 µM each of a forward primer (CTACCAGCATTCACGGTGAC: SEQ ID NO: 3) and a reverse primer (GTAGCAGGTCCGCTGAACGC: SEQ ID NO: 4). After this reaction solution was incubated for 3 minutes at 94°C using a thermal cycler, 35 cycles consisting of 15 seconds at 94°C, 15 seconds at 62°C, and 30 seconds at 72°C were repeated, and finally, it was incubated for 3 minutes at 72°C.

As an internal standard, expression of the gsdf gene in each host was examined by PCR. cDNA synthesized from total RNA of the gonads of each host were used as templates, and 10 µL of reaction solution was prepared, containing each template as well as 2.5 units of TaKaRa Ex Taq (Takara Bio Inc.), 1 µL of 10X Ex Taq Buffer, 0.2 mM of dNTP Mixture, and 1 µM each of a forward primer (GCTCTCAACTTGCAGGCTGA: SEQ ID NO: 5) and a reverse primer (CCCAGCCCAGATCTTTCATG: SEQ ID NO: 6). After this reaction solution was incubated for 3 minutes at 94°C using a thermal cycler, 30 cycles consisting of 30 seconds at 94°C, 30 seconds at 58°C, and 30 seconds at 72°C were repeated, and finally, it was incubated for 3 minutes at 72°C.

As a result, a 343 bp Pacific bluefin tuna vasa gene sequence (accession no. EU253482, 1516-1858) was detected in 8 (20%) of the 40 blue mackerel-chub mackerel hybrids, in 0 (0%) of the 19 diploid chub mackerel, and in 2 (15%) of the 13 triploid chub mackerel. Furthermore, a 217 bp chub mackerel gsdf gene sequence (accession no. GQ404694, 5-221) was detected in all individuals of the blue mackerel hybrid hosts, chub mackerel diploid hosts, and chub mackerel triploid hosts, demonstrating that RNA was extracted without problems from the gonads of these individuals.

These results suggest that the gonads of the blue mackerel-chub mackerel hybrids and chub mackerel triploids possessed Pacific bluefin tuna reproductive cells for 5 months. The incidence thereof was higher in the blue mackerel-chub mackerel hybrids than in the chub mackerel triploids. Since the blue mackerel-chub mackerel hybrid is highly likely to possess Pacific bluefin tuna reproductive cells, it is expected that Pacific bluefin tuna gametes will be obtained in the blue mackerel-chub mackerel hybrid, that Pacific bluefin tuna fertilized eggs will be obtained by, for example, artificially inseminating the obtained gametes, and that Pacific bluefin tuna adult fish will be obtained from the obtained Pacific bluefin tuna fertilized eggs.

Thus, the blue mackerel-chub mackerel hybrid is advantageous as a Pacific bluefin tuna recipient.

### Example 3

### (1) Production of Euthynnus crossbreed

As a hybrid, a Euthynnus crossbred of Euthynnus affinis and Euthynnus alletteratus, that is, a Euthynnus affinis-Euthynnus alletteratus hybrid, is produced by artificial fertilization using unfertilized eggs obtained by squeezing mature female Euthynnus affinis and sperm harvested from mature male Euthynnus alletteratus. The fertilized eggs of the obtained Euthynnus affinis-Euthynnus alletteratus hybrid are reared in 17°C to 18°C seawater.

The presence or absence of reproductive cells and the degree of maturation of the gonads is ascertained for the obtained Euthynnus affinis-Euthynnus alletteratus hybrid using techniques such as RT-PCR and in situ hybridization.

The presence of reproductive cells is no longer ascertained in the obtained Euthynnus affinis-Euthynnus alletteratus hybrid at approximately 60 days of age. At approximately 120 days of age, the gonads exhibit a testicle-like or ovary-like shape, but the presence of reproductive cells is not ascertained. This Euthynnus affinis-Euthynnus alletteratus hybrid grows to a body length of 30 to 60 cm and a body weight of 1 to 5 g in approximately 2 years, but it is ascertained that the Euthynnus affinis-Euthynnus alletteratus hybrid has immature gonads without reproductive cells and is sterile.

### (2) Transplantation of Pacific bluefin tuna primordial germ cells into Euthynnus affinis-Euthynnus alletteratus hybrid

As donor cells, dispersed cells harvested from testicles of a 30 kg immature male Pacific bluefin tuna are used. Pacific bluefin tuna testicles harvested from Pacific bluefin tuna and cooled with ice overnight are cut into 1 mm squares, and reacted in L-15 medium "Leibovitz's L-15" (Life Technologies Corp.) containing collagenase (2 mg/mL), Dispase II (500 U/mL), fetal bovine serum (5 v/v%), and DNase (100 U/mL), and a cell suspension is obtained. The cell membranes of cells contained in this suspension are stained with red fluorescent dye PKH26 and then transplant into the peritoneum of hosts using an injector in a quantity of 10000 to 100000 cells per host.

Euthynnus affinis-Euthynnus alletteratus hybrid larvae 10 days after hatching are used as hosts. Transplantation is carried out in 30 to 50 Euthynnus affinis-Euthynnus alletteratus hybrid larvae. After transplantation, the larvae are reared in 23°C to 28°C seawater.

At 1 to 10 months after transplantation, it is ascertained that the gonads of Euthynnus affinis-Euthynnus alletteratus hybrid larvae possess Pacific bluefin tuna reproductive cells. At 12 to 72 months after transplantation, Pacific bluefin tuna gametes are obtained in the Euthynnus affinis-Euthynnus alletteratus hybrid.

### Example 4

A Euthynnus affinis-Euthynnus lineatus (black skipjack tuna) hybrid is obtained by artificial fertilization in the similar manner as Example 3(1) except that Euthynnus lineatus is used instead of Euthynnus alletteratus. The Euthynnus affinis-Euthynnus lineatus hybrid has immature gonads without reproductive cells and is sterile.

Similar to Example 3(2), sperm obtained from Pacific bluefin tuna testicles are transplanted into the peritoneum of sterile Euthynnus affinis-Euthynnus lineatus hybrid larvae 10 days after hatching using an injector and are reared. At 1 to 10 months after transplantation, the gonads of Euthynnus affinis-Euthynnus lineatus hybrid larvae possess Pacific bluefin tuna reproductive cells. At 12 to 72 months after transplantation, Pacific bluefin tuna gametes are obtained from the gonads of the Euthynnus affinis-Euthynnus lineatus hybrid.

### Example 5

A Euthynnus affinis-bonito hybrid is obtained by artificial fertilization in the similar manner as Example 3(1) except that Euthynnus affinis and bonito are used instead of the combination of Euthynnus affinis and Euthynnus alletteratus. The Euthynnus affinis-bonito hybrid is ascertained to have immature gonads without reproductive cells and to be sterile.

Similar to Example 3(2), sperm obtained from Pacific bluefin tuna testicles are transplanted into the peritoneum of sterile Euthynnus affinis-bonito hybrid larvae 10 days after hatching using an injector and are reared. At 1 to 10 months after transplantation, the gonads of Euthynnus affinis-bonito hybrid larvae possess Pacific bluefin tuna reproductive cells. At 12 to 72 months after transplantation, Pacific bluefin tuna gametes are obtained in the Euthynnus affinis-bonito hybrid.

## Claims

1. A transplant fish obtainable by a transplant fish production method comprising:
selecting a donor fish species;
selecting first and second fish species;
producing a sterile hybrid fish species of the selected first fish species and second fish species; and
transplanting reproductive cells of the donor fish species into the produced sterile hybrid fish species;
wherein the donor fish species is selected from the Scombridae family;
and wherein the first and second fish species are selected from species different from the donor fish species among species selected from the Sardini tribe, such as the Gymnosarda genus and Sarda genus; the
Scomberomorini tribe, such as the Acanthocybium genus and
Scomberomorus genus; the Scombrini tribe, such as the Scomber genus and Rastrelliger genus; and the Thunnini tribe, such as the Auxis genus, Euthynnus genus, Katsuwonus genus, and Thunnus genus.

2. The transplant fish according to claim 1, wherein the first fish species and the second fish species are both of the Thunnus genus.

3. The transplant fish according to claim 1, wherein the first fish species and the second fish species are both of the Euthynnus genus.

## Patentansprüche

1. Transplantationsfisch, erhältlich durch ein Verfahren zur Herstellung eines Transplantationsfisches, das umfasst:
Wählen einer Spenderfischart;
Wählen einer ersten und einer zweiten Fischart;
Erzeugen einer sterilen Hybridfischart aus der gewählten ersten Fischart und zweiten Fischart; und
Transplantieren von Keimzellen der Spenderfischart in die erzeugte sterile Hybridfischart;
wobei die Spenderfischart aus der Familie Scombridae ausgewählt wird und wobei die erste und die zweite Fischart aus Arten ausgewählt werden, die sich von der Spenderfischart unterscheiden, aus Arten ausgewählt aus dem Stamm Sardini, wie die Gattung Gymnosarda und die Gattung Sarda; dem Stamm Scomberomorini, wie die Gattung Acanthocybium und die Gattung Scomberomorus; dem
Stamm Scombrini, wie die Gattung Scomber und die Gattung Rastrelliger; und
dem Stamm Thunnini, wie die Gattung Auxis, die Gattung Euthynnus, die Gattung Katsuwonus und die Gattung Thunnus.

2. Transplantationsfisch nach Anspruch 1, wobei die erste Fischart und die zweite Fischart beide der Gattung Thunnus angehören.

3. Transplantationsfisch nach Anspruch 1, wobei die erste Fischart und die zweite Fischart beide der Gattung Euthynnus angehören.

## Revendications

1. Poisson transplanté pouvant être obtenu par un procédé de production de poisson transplanté comprenant les étapes consistant à :
sélectionner une espèce de poisson donneur ;
sélectionner des première et seconde espèces de poissons ;
produire une espèce de poisson hybride stérile de la première espèce de poisson et de la seconde espèce de poisson sélectionnées ; et
transplanter des cellules reproductrices de l'espèce de poisson donneur dans l'espèce de poisson hybride stérile produite ;
dans lequel l'espèce de poisson donneur est sélectionnée parmi la famille des Scombridae ;
et dans lequel les première et seconde espèces de poisson sont sélectionnées parmi des espèces différentes de l'espèce de poisson donneur parmi des espèces sélectionnées parmi la tribu des Sardini, telle que le genre Gymnosarda et le genre Sarda ; la tribu des Scomberomorini, telle que le genre Acanthocybium et le genre Scomberomorus ; la tribu des Scombrini, telle que le genre Scomber et le genre Rastrelliger ; et la tribu des Thunnini, telle que le genre Auxis, le genre Euthynnus, le genre Katsuwonus et le genre Thunnus.

2. Poisson transplanté selon la revendication 1, dans lequel la première espèce de poisson et la seconde espèce de poisson sont toutes les deux du genre Thunnus.

3. Poisson transplanté selon la revendication 1, dans lequel la première espèce de poisson et la seconde espèce de poisson sont toutes les deux du genre Euthynnus.
